# EUROPEAN PATENT SPECIFICATION

(11) **EP 1 281 421 B1**
(45) Date of publication and mention of the grant of the patent: **07.05.2008**
(21) Application number: 02078021.9
(22) Date of filing: 24.07.2002
(51) Int. Cl.: B01D 11/02, A61K 8/00, B01J 8/00

(54) **Apparatus for producing cosmetic products**
Vorrichtung zur Herstellung von kosmetischen Produkten
Dispositif pour la préparation des produits cosmétiques

(30) Priority: 02.08.2001 IT MI20011695
(43) Date of publication of application: 05.02.2003
(73) Proprietor: Intercos S.p.A., 20122 Milano (IT)
(72) Inventor: Maio, Giuseppe, 20080 Zelo Surrigone MI (IT); Mandelli, Antonio, 20063 Cernusco Sul Naviglio MI (IT)
(74) Representative: Mittler, Enrico

(56) References cited:
- EP-A- 0 590 647
- EP-A- 1 197 513
- US-A- 5 833 891
- US-A- 6 114 414
- US-B1- 6 241 382

## Description

The present invention refers to an apparatus for producing cosmetic products.

Cosmetic products obtained from the mixture of a powder phase with a lipidic, oily or polymeric phase in the presence of a solvent are already known. The mixture may have the aim of treating the powder by improving its characteristics and is carried out in mills of various types where the two phases are mixed for a variable time (sometimes for a long time) to obtain a homogeneous product.

The JP patent 11-47681 describes a method for coating fine particles using a supercritical fluid as the solvent. In an apparatus that uses the above method, the liquid carbon dioxide fed from a tank is conveyed through a valve to a preheating column installed in a controlled-temperature water bath. The carbon dioxide is heated in the latter at a temperature that is greater than the equilibrium temperature of the carbon dioxide, so as to obtain a supercritical fluid that is conveyed to an extraction cell. The latter contains a high molecular weight compound, for example PEG, provided as solute, a solvent, for example ethanol, that enables the high molecular compound to be dissolved in the carbon dioxide in supercritical condition, and fine particles made of at least one non-organic material, such as titanium dioxide. All the above substances are completely dissolved in the carbon dioxide in supercritical condition used as solvent. Then the carbon dioxide in supercritical condition in which the above solute is dissolved is introduced into a controlled-temperature air bath through a heated tube and sprayed by means of nozzles contained therein. In this manner the carbon dioxide in supercritical condition expands and its capacity to dissolve the solute decreases. The final result is constituted of fine particles of titanium dioxide coated with PEG collected by a panel.

In view of the state of the technique described, the object of the present invention is to produce an apparatus for the production of cosmetic products that use carbon dioxide in non-supercritical condition for dissolving fatty substances.

In accordance with the present invention this object is reached by means of an apparatus as defined in claim 1.

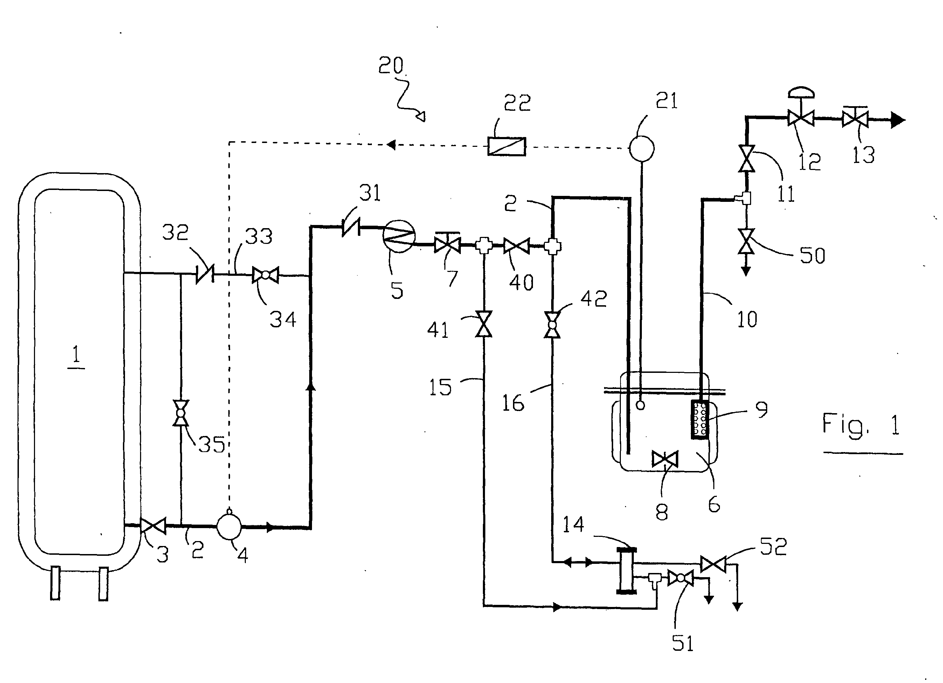

The characteristics and advantages of the present invention will be evident from the following detailed description of an embodiment thereof, illustrated as non-limiting example in the drawing of Figure 1 in which a schematic view of the apparatus for producing cosmetic products according to the present invention is shown.

The apparatus according to the invention shown in Figure 1 comprises a tank 1 containing carbon dioxide in the liquid state, for example at a pressure of approximately 20 bar and at a temperature of approximately - 20°C. The carbon dioxide in the liquid state and in non super-critical conditions leaves the tank 1 through a duct 2 fitted with a high-pressure valve 3. The liquid carbon dioxide in non super-critical conditions enters a pump 4 capable of increasing its pressure and conveying it to a carbon dioxide/water heat exchanger 5. The liquid obtained is sent to a reactor 6 through a micrometric valve 7 that permits the initial slow passage of the liquid to the reactor 6; in this manner the pressure of the liquid in the reactor 6 reaches 20 bar without provoking rapid expansion of the carbon dioxide which would cause it to freeze. After the pressure in the reactor has reached 20 bar pump 4 is activated.

The reactor 6 contains a mechanical agitator 8 that enables the cosmetic powders and lipidic substances that have been previously added to be mixed with the liquid carbon dioxide in non super-critical conditions which acts as solvent; under working conditions a pressure of for example approximately 60 bar and a temperature of between 20°C and 25°C will be reached in the reactor 6.

At the end of mixing the carbon dioxide is filtered by a special filter 9 and is discharged externally through a pipe 10 and special valves 11, 12 and 13, of which the valve 12 can be regulated in pressure and opens only at a pressure which is greater than that preset and the valve 13 is an air valve with manual opening.

The apparatus comprises a small test container 14 connected to the duct 2 upstream from the reactor 6 by means of two ducts 15 and 16 connected to the same duct 2 and each fitted with a valve 41 and a valve 42 respectively; in duct 2 a valve 40 is present between ducts 15 and 16. The container 14 enables the color of a sample of the product present in the reactor 6 to be tested and to make corrections if the product is not that desired. Air valves 51 and 52 are associated with container 14.

The apparatus comprises a device 20 for stopping the pump 4, comprising a level sensor 21 which, when the liquid present in the reactor 6 has reached a preset level, activates a relay 22 that commands pump 4 to stop.

Check valves 31 and 32 are also provided; more specifically, a check valve 31 of the carbon dioxide in the liquid phase into the tank 1 is provided directly in the duct 2 positioned upstream from the exchanger 5 and downstream from the pump 4, and a check valve 32 of the carbon dioxide in gaseous phase into the tank 1, positioned in a pipe 33 connected in derivation to the duct 2 by means of a valve 34. The check valve 32 is connected to the tank 1, and also to the duct 2 between the valve 3 and the pump 4 by means of a valve 35.

The apparatus in Figure 1 functions as follows.

The liquid carbon dioxide in non super-critical conditions flows out of tank 1 and is conveyed by means of the duct 2 towards the heat exchanger 5. The liquid obtained is sent to the reactor 6 through the micrometric valve 7, which permits the liquid to pass slowly initially so that its pressure in the reactor 6 reaches 20 bar without provoking rapid expansion of the carbon dioxide which would cause it to freeze. When the 20 bar have been reached inside the reactor 6, which means reaching the pressure of equilibrium between reactor 6 and tank 1, pump 4 is activated to send under pressure the liquid with liquid carbon dioxide in non super-critical conditions to the reactor 6. When the preset level of liquid inside the reactor 6 is reached, the device 20 stops the pump in the manner previously described. The liquid carbon dioxide in non super-critical conditions serves as a solvent for lipidic substances and other powder and non-powder ingredients previously poured in the reactor 6; the agitator 8 mixes the substances and the fluid.

When the mixing is completed the carbon dioxide will be discharged externally by means of duct 10 and the valves 11, 12 and 13.

During the preparation of the compound a phase for testing a sample of the product made inside the reactor 6 is provided for. This occurs prior to the closing of the valve 7 and return of the product through duct 2. The valve 40 positioned in duct 2 is opened, as is the valve 41 while the valve 42 remains closed; the product of the reactor 6 flows in duct 15 and reaches the container 14. Once the valve 52 is opened to reduce the pressure in the container 14 the latter can be opened to verify the color of the product and to make suitable corrections if necessary (addition of color or other substances). The sample of the corrected product is conveyed to the reactor 6 by means of duct 16, once the valves 52 and 40 are closed and the valves 7 and 42 opened. The valve 40 is then opened again and the valves 41 and 42 closed to restore the previous working condition.

Once the preparation of the product in the reactor 6 has been completed, the carbon dioxide can be let out through the vent connections consisting of the duct 10 and the open pin valve 50, while the valve 7 remains closed. The reactor 6 can then be opened to discharge the cosmetic product thus obtained, which can be the final product or a coating of some type.

## Claims

1. Apparatus for producing cosmetic products comprising a reactor (6) provided with an agitator (8) suitable for mixing lipidic substances and powders in the presence of solvent to obtain a mixed cosmetic product, means (1, 2-5, 7, 31-35) for feeding into said reactor (6) carbon dioxide in a liquid phase for use as solvent for said lipidic substances and powders, and means (9-13) for discharging carbon dioxide outside said reactor (6), **characterized in that** said carbon dioxide is fed in non super-critical conditions and there is provided a test container (14) for carrying out a test on a sample of the cosmetic product mixed into the reactor (6) and, if necessary, to add substances to correct the color or other, said test container (14) being connected to said reactor (6) by duct and valve means (2,15,16, 40-42) for feeding said container (14) with said sample and for returning the corrected sample to the reactor (6).

2. Apparatus according to claim 1, **characterized in that** said duct and valve means (2, 15, 16, 40-42) comprise ducts (2, 15, 16) fitted with valves (7, 40-42) that are alternately opened and closed to cause conveying of the product sample from the reactor (6) to the container (14) and the return of the corrected sample from the test container (14) to the reactor (6).

3. Apparatus according to claim 1, **characterized in that** said means (1,2-5, 7, 31-35) for feeding the reactor with carbon dioxide in a liquid phase in non super-critical conditions comprise a tank (1) containing said carbon dioxide in a liquid phase in non super-critical conditions, a valve (7) that permits the initial slow passage of said carbon dioxide in a liquid phase in non super-critical conditions from the tank (1) into the reactor (6) so as to avoid freezing due to an excessive sudden variation in pressure.

4. Apparatus according to claim 3, **characterized in that** said preset pressure inside the reactor (6) must balance that inside the tank (1).

5. Apparatus according to claim 4 3, **characterized in that** said means (1, 2-5, 7, 31-35) for feeding the reactor with carbon dioxide in a liquid phase in non super-critical conditions comprise a pump (4) that is activated once the equilibrium pressure between reactor (6) and tank (1) is reached.

6. Apparatus according to claim 3, **characterized in that** it comprises a device (20) for stopping the pump (4), said device (20) comprising a level sensor (21) which upon reaching a preset level of the mixture in the reactor (6) commands the pump (4) to stop.

7. Apparatus according to claim 3, **characterized in that** said means (2-5, 7, 31-35) for feeding the reactor (6) with carbon dioxide in a liquid phase in non super-critical conditions comprises a carbon dioxide/water heat exchanger (5) upstream of said valve (7).

8. Apparatus according to claim 7, **characterized in that** said means (2-5, 7, 31-35) for feeding the reactor (6) with carbon dioxide in a liquid phase in non super-critical conditions comprises check valves (31, 32) of the carbon dioxide in a liquid phase and in a gassy phase positioned upstream of said exchanger (5).

9. Apparatus according to claim 1, **characterized in that** said means (9-13) for discharging carbon dioxide outside said reactor (6) comprises a filter (9) inside the reactor (6) associated to a pipe (10) that leads to a pressure valve (12) external to the reactor and to an air valve (13).

10. Apparatus according to claim 9, **characterized in that** said pressure valve (12) is regulated by a preset pressure.

11. Apparatus according to claim 1, **characterized in that** it comprises vent connections (9,10,50; 2, 42, 16, 14, 52; 2, 40, 41, 15, 51) of the carbon dioxide.

## Patentansprüche

1. Vorrichtung zum Erzeugen kosmetischer Produkte mit:
einem Reaktor (6), der mit einem Rührwerk (8) versehen ist, das geeignet ist, lipidische Substanzen und Pulver in der Gegenwart eines Lösungsmittels zu mischen zum Gewinnen eines gemischten kosmetischen Produkts,
einem Mittel (1, 2-5, 7, 31-35) zum Zuführen von Kohlendioxid in flüssiger Phase in den Reaktor (6) zur Verwendung als Lösungsmittel für die lipidischen Substanzen und Pulver, und
ein Mittel (9-13) zum Ablassen von Kohlendioxid aus dem Reaktor (6),
**dadurch gekennzeichnet, dass**
das Kohlendioxid in einem nicht überkritischen Zustand zugeführt wird und
ein Testbehälter (14) bereitgestellt ist, um einen Test an einer Probe des in dem Reaktor (6) gemischten kosmetischen Produkts durchzuführen und erforderlichenfalls Substanzen hinzuzufügen, um die Farbe und Anderes zu ändern,
wobei der Testbehälter (14) mit dem Reaktor (6) verbunden ist über ein Leitungs-und-Ventil-Mittel (2, 15, 16, 40-42) zum Zuführen der Probe zu dem Behälter (14) und zum Zurückführen der korrigierten Probe zu dem Reaktor (6).

2. Vorrichtung nach Anspruch 1, **dadurch gekennzeichnet, dass** das Leitungs-und-Ventil-Mittel (2, 15, 16, 40-42) Leitungen (2, 15, 16) enthält, die mit Ventilen (7, 40-42) versehen sind, die abwechselnd geöffnet und geschlossen werden zum Bewirken der Förderung der Produktprobe von dem Reaktor (6) in den Behälter (14) und des Zurückführens der korrigierten Probe von dem Testbehälter (14) zu dem Reaktor (6).

3. Vorrichtung nach Anspruch 1, **dadurch gekennzeichnet, dass** das Mittel (1, 2-5, 7, 31-35) zum Zuführen von Kohlendioxid in flüssiger Phase im nicht überkritischen Zustand zu dem Reaktor enthält:
einen Tank (1), der das Kohlendioxid in flüssiger Phase im nicht überkritischen Zustand enthält, und
ein Ventil (7), das den anfänglichen langsamen Duxchtritt des Kohlendioxids in flüssiger Phase im nicht überkritischen Zustand von dem Tank (1) in den Reaktor (6) ermöglicht zum Vermeiden eines Gefrierens aufgrund einer übermäßigen plötzlichen Druckänderung.

4. Vorrichtung nach Anspruch 3, **dadurch gekennzeichnet, dass** der voreingestellte Druck in dem Reaktor (6) mit demjenigen im Tank (1) im Gleichgewicht sein muss.

5. Vorrichtung nach Anspruch 3, **dadurch gekennzeichnet, dass** das Mittel (1, 2-5, 7, 31-35) zum Zuführen von Kohlendioxid in flüssiger Phase im nicht überkritischen Zustand zu dem Reaktor eine Pumpe (4) enthält, die aktiviert wird, wenn das Druckgleichgewicht zwischen Reaktor (6) und Tank (1) erreicht ist.

6. Vorrichtung nach Anspruch 3, **dadurch gekennzeichnet, dass**
sie eine Vorrichtung (20) zum Anhalten der Pumpe (4) enthält,
wobei die Vorrichtung (20) einen Pegelsensor (21) enthält, der bei Erreichen eines vorbestimmten Pegels der Mischung in dem Reaktor (6) die Pumpe (4) anweist, anzuhalten.

7. Vorrichtung nach Anspruch 3, **dadurch gekennzeichnet, dass** das Mittel (2-5, 7, 31-35) zum Zuführen von Kohlendioxid in flüssiger Phase im nicht überkritischen Zustand zu dem Reaktor (6) einen Kohlendioxid/Nasser-Wärmetauscher (5) stromaufwärts des Ventils (7) enthält.

8. Vorrichtung nach Anspruch 7, **dadurch gekennzeichnet, dass** das Mittel (2-5, 7, 31-35) zum Zuführen von Kohlendioxid in flüssiger Phase im nicht. überkritischen Zustand zu dem Reaktor (6) Sperrventile (31, 32) für Kohlendioxid in flüssiger Phase und in gasförmiger Phase enthält, die stromaufwärts von dem Wärmetauscher (5) angeordnet sind.

9. Vorrichtung nach Anspruch 1, **dadurch gekennzeichnet, dass** das Mittel (9-13) zum Ablassen von Kohlendioxid aus dem Reaktor (6) ein Filter (9) in dem Reaktor (6) enthält, die einer Leitung (10) zugeordnet ist, die zu einem Druckventil (12) außerhalb des Reaktors und zu einem Luftventil (13) führt.

10. Vorrichtung nach Anspruch 9, **dadurch gekennzeichnet, dass** das Druckventil (12) durch einen voreingestellten Druck reguliert wird.

11. Vorrichtung nach Anspruch 1, **dadurch gekennzeichnet, dass** sie Entlüftungsverbindungen (9, 10, 50; 2, 42, 16, 14, 52; 2, 40, 41, 15, 51) für das Kohlendioxid enthält.

## Revendications

1. Appareil pour produire des produits cosmétiques comprenant un réacteur (6) doté d'un agitateur (8) approprié pour mélanger des substances et des poudres lipidiques en présence de solvant pour obtenir un produit cosmétique mélangé, des moyens (1, 2-5, 7, 31-35) pour l'alimentation dans ledit réacteur (6) du dioxyde de carbone dans une phase liquide destiné à être utilisé en tant que solvant, pour lesdites substances et poudres lipidiques, et des moyens (9-13) pour décharger le dioxyde de carbone à l'extérieur dudit réacteur (6),
**caractérisé en ce que** ledit dioxyde de carbone est alimenté dans des conditions qui ne sont pas supercritiques et **en ce que** l'on propose un récipient d'essai (14) pour réaliser un essai sur un échantillon du produit cosmétique mélangé dans le réacteur (6) et, si nécessaire, pour ajouter des substances afin de corriger la couleur ou autre, ledit récipient d'essai (14) étant raccordé audit réacteur (6) par des moyens de conduit et de soupape (2, 15, 16, 40-42) pour alimenter ledit récipient (14) avec ledit échantillon et pour faire revenir l'échantillon corrigé dans le réacteur (6).

2. Appareil selon la revendication 1, **caractérisé en ce que** lesdits moyens de conduit et de soupape (2, 15, 16, 40-42) comprennent des conduits (2, 15, 16) équipés de soupapes (7, 40-42) qui s'ouvrent et se ferment de manière alternée pour provoquer le transport de l'échantillon de produit du réacteur (6) jusqu'au récipient (14) et le retour de l'échantillon corrigé du récipient d'essai (14) dans le réacteur (6).

3. Appareil selon la revendication 1, **caractérisé en ce que** lesdits moyens (1, 2-5, 7, 31-35) pour alimenter le réacteur avec du dioxyde de carbone dans une phase liquide dans des conditions qui ne sont pas supercritiques comprennent un réservoir (1) contenant ledit dioxyde de carbone dans une phase liquide dans des conditions qui ne sont supercritiques, une soupape (7) qui permet le passage lent initial dudit dioxyde de carbone dans une phase liquide dans des conditions qui ne sont pas supercritiques du réservoir (1) dans le réacteur (6) afin d'éviter le gel dû à une variation subite excessive de pression.

4. Appareil selon la revendication 3, **caractérisé en ce que** ladite pression prédéterminée à l'intérieur du réacteur (6) doit équilibrer celle qu'il y a à l'intérieur du réservoir (1).

5. Appareil selon la revendication 3, **caractérisé en ce que** lesdits moyens (1, 2-5, 7, 31-35) pour alimenter le réacteur avec du dioxyde de carbone dans une phase liquide dans des conditions qui ne sont pas supercritiques comprennent une pompe (4) qui est activée une fois que la pression d'équilibre entre le réacteur (6) et le réservoir (1) est atteinte

6. Appareil selon la revendication 3, **caractérisé en ce qu'**il comprend un dispositif (20) pour arrêter la pompe (4), ledit dispositif (20) comprenant un capteur de niveau (21) qui après avoir atteint un niveau prédéterminé du mélange dans le réacteur (6), commande l'arrêt de la pompe (4).

7. Appareil selon la revendication 3, **caractérisé en ce que** lesdits moyens (2-5, 7, 31-35) pour alimenter le réacteur (6) avec du dioxyde de carbone dans une phase liquide dans des conditions qui ne sont pas supercritiques, comprennent un échangeur de chaleur de dioxyde de carbone/eau (5) en amont de ladite soupape (7).

8. Appareil selon la revendication 7, **caractérisé en ce que** lesdits moyens (2-5, 7, 31-35) pour alimenter le réacteur (6) avec du dioxyde de carbone dans une phase liquide dans des conditions qui ne sont pas supercritiques comprennent des soupapes anti-retour (31, 32) de dioxyde de carbone dans une phase liquide et une phase gazeuse positionnée en amont dudit échangeur (5).

9. Appareil selon la revendication 1, **caractérisé en ce que** lesdits moyens (9-13) pour décharger le dioxyde de carbone à l'extérieur dudit réacteur (6) comprennent un filtre (9) à l'intérieur du réacteur (6) associé à un tuyau (10) qui conduit jusqu'à une soupape de pression (12) à l'extérieur du réacteur et une soupape d'air (13),

10. Appareil selon la revendication 9, **caractérisé en ce que** ladite soupape de pression (12) est régulée selon une pression prédéterminée"

11. Appareil selon la revendication 1, **caractérisé en ce qu'**il comprend des raccordements d'aération (9, 10, 50; 2, 42, 16, 14, 52 ; 2, 40, 41, 15, 51) de dioxyde de carbone.
